Europäisches Patentamt

European Patent Office

Office européen des brevets

(19)

(11) **EP 0 535 578 B1**

(12) **EUROPÄISCHE PATENTSCHRIFT**

(45) Veröffentlichungstag und Bekanntmachung des Hinweises auf die Patenterteilung:
**14.05.1997 Patentblatt 1997/20**

(51) Int Cl.⁶: **C12M 1/40**

(21) Anmeldenummer: **92116580.9**

(22) Anmeldetag: **28.09.1992**

(54) **Dickschicht-Leitfähigkeitselektroden als Biosensor**

Thick layer conductivity electrodes as biosensor

Electrodes de conductivité à couche épaisse comme capteur biologique

(84) Benannte Vertragsstaaten:
**CH DE FR GB IT LI NL**

(30) Priorität: **28.09.1991 DE 4132441**

(43) Veröffentlichungstag der Anmeldung:
**07.04.1993 Patentblatt 1993/14**

(73) Patentinhaber:
• **Gesellschaft für Biotechnologische Forschung mbH (GBF)**
**D-38124 Braunschweig (DE)**
• **SIEGERT electronic GmbH**
**90556 Cadolzburg (DE)**

(72) Erfinder:
• **Bilitewski, Ursula, Dr.**
**W-3300 Braunschweig (DE)**
• **Drewes, Wiebke**
**W-3300 Braunschweig (DE)**
• **Bechtold, Franz**
**W-8501 Cadolzburg 1 (DE)**

(74) Vertreter: **Boeters, Hans Dietrich, Dr. et al**
**Patentanwälte Boeters & Bauer,**
**Bereiteranger 15**
**81541 München (DE)**

(56) Entgegenhaltungen:
• **PATENT ABSTRACTS OF JAPAN, unexamined applications, P Sektion, Band 14, Nr. 405, 31. August 1990 THE PATENT OFFICE JAPANESE GOVERNMENT Seite 131 P 1100**
• **PATENT ABSTRACTS OF JAPAN, unexamined applications, P Sektion, Band 14, Nr. 405, 31. August 1990 THE PATENT OFFICE JAPANESE GOVERNMENT Seite 132 P 1100**
• **PATENT ABSTRACTS OF JAPAN, unexamined applications, C Sektion, Band 13, Nr. 169, 21. April 1989 THE PATENT OFFICE JAPANESE GOVERNMENT Seite 10 C 587**
• **PATENT ABSTRACTS OF JAPAN, unexamined applications, P Sektion, Band 12, Nr. 199, 09. Juni 1988 THE PATENT OFFICE JAPANESE GOVERNMENT Seite 144 P 714**
• **PATENT ABSTRACTS OF JAPAN, unexamined applications, P Sektion, Band 12, Nr. 399, 24. Oktober 1988 THE PATENT OFFICE JAPANESE GOVERNMENT Seite 81 P 775**
• **PATENT ABSTRACTS OF JAPAN, unexamined applications, P Sektion, Band 12, Nr. 399, 24. Oktober 1988 THE PATENT OFFICE JAPANESE GOVERNMENT Seite 83 P 775**
• **PATENT ABSTRACTS OF JAPAN, unexamined applications, P Sektion, Band 13, Nr. 294, 07. July 1989 THE PATENT OFFICE JAPANESE GOVERNMENT Seite 148 P 894**

**Beschreibung**

Die vorliegende Erfindung befaßt sich mit Dickschicht-Leitfähigkeitselektroden als Biosensor, insbesondere mit Biosensoren für Leitfähigkeitsmessungen verschiedener Analyte.

In den letzten Jahren wurde die Entwicklung von Biosensoren auf dem Gebiet der Forschung stark vorangetrieben, insbesondere bei der gezielten Erkennung bestimmter Stoffe aus einer komplexen Probenmatrix kommt der Verwendung der Biosensoren zunehmend Bedeutung zu. Biomoleküle, wie beispielsweise Enzyme, Antikörper, Nukleinsäuren oder ganze Organismen, reagieren dabei spezifisch mit dem zu detektierenden Analyten. Die sich bei der Reaktion der biologischen Komponenten mit dem Analyten ändernden Parameter werden für die Signalverarbeitung über einen Transducer (z.B. Elektroden, Feldeffekt-Transistoren, Photometer oder Glasfaseroptiken) in strierbare Signale gewandelt.

Biosensoren werden häufig als Eintauchelektroden für Einmalbestimmungen hergestellt. Biosensoren nach dem Oberbegriff des Anspruchs 1 sind seit längerem als Leitfähigkeits-Biosensoren auf der Basis sog. Dünnschicht-Elektroden bekannt. In Materialien dieser Dünnschicht-Elektroden sind in der Regel Gold, Platin, Palladium oder Legierungen zwischen diesen Metallen mit Silber. Diese Dünnschicht-Leitfähigkeits-Biosensoren haben den Nachteil, daß sie in einer verhältnismäßig aufwendigen Technik hergestellt werden müssen, die sehr kostenintensiv ist, und somit den eigentlichen Biosensor für einen weitverbreiteten Einsatz zu kostspielig macht. Ferner wird als nachteilig angesehen, daß die Herstellung des Transducers nicht mit der gleichen Technik erfolgen kann, wie das Aufbringen einer Enzymschicht. Darüber hinaus ist eine extreme Miniaturisierung in vielen Fällen nicht notwendig und wird wegen einer besseren Handhabbarkeit der Sensoren auch nicht immer angestrebt.

In jüngerer Zeit werden mehr und mehr verschiedene Arten von Dickschicht-Techniken zur Herstellung von leitfähigen Strukturen auf einem Substrat, was in der Regel aus Keramik besteht, angewendet. Die Dickschicht-Technik beruht darauf, daß auf einem Substrat im Siebdruckverfahren Pasten aufgedruckt und eingebrannt werden. Auf diese Weise lassen sich Hybrid-Schaltungen herstellen, da die Pasten bestimmte elektronische Eigenschaften (Leitfähigkeit, Kapazität) besitzen. In jüngster Zeit wird diese Technik auch für die Herstellung von Sensoren verschiedenster Art eingesetzt. Für die Herstellung von Biosensoren sind vor allem die elektrochemischen Sensoren von Bedeutung. Da es eine Vielzahl von käuflichen Pasten unterschiedlichster Zusammensetzung und Leitfähigkeit gibt, lassen sich Strukturen drucken, die vom Prinzip her wie konventionelle Edelmetallelektroden (Gold, Platin, Palladium und Legierungen zwischen diesen Metallen mit Silber) eingesetzt werden können. Diese Elektroden haben den Vorteil, daß sie verhältnismäßig geringe Abmessungen aufweisen, die typischerweise ca. 50 µm für die Linienbreite und ca. 10 µm für die Schichtdicke aufweisen. Diese Vorrichtungen können in einem industriellen Prozess gefertigt werden und stehen damit als Massenartikel für den Verbraucher zur Verfügung.

Leitfähigkeitsmessungen oder sog. Admittanzmessungen beruhen auf der Messung des Stromes als Funktion einer angelegten Spannung, wobei in der Regel Wechselspannung mit einer typischen Amplitude von 1V und einer Frequenz von 4kHz verwendet werden. Die Leitfähigkeit Y einer Lösung ist der Kehrwert des Widerstandes R und damit

$$Y = I/U.$$

Sie wird in der Regel als komplexe Größe dargestellt:

$$Y = G + iB.$$

Hierbei ist der Realteil G der ohmsche Anteil, d.h. der Anteil, der durch die Wanderung der Ladungen im elektrischen Feld bestimmt wird. Zu diesem Anteil kommen noch im wesentlichen kapazitive Anteile hinzu, die durch die Grenzschicht Elektrode/Lösung bestimmt werden und zu einer Phasenverschiebung zwischen angelegter Spannung und resultierendem Strom führen. Sie werden als Blindleitwert B bezeichnet und stellen die imaginäre Komponente der Admittanz Y dar. Außer diesen Beiträgen hängt der Meßwert auch noch von der Fläche A und dem Abstand 1 der verwendeten Elektroden ab, die in der Zellkonstante mit 1/A zusammengefaßt werden. Herkömmliche higkeitsmeßzellen enthalten daher zwei Elektroden definierter Fläche, die in einem bestimmten Abstand zueinander stehen. Leitfähigkeitselektroden können auch die Grundlage von Biosensoren bilden, da sich bei einer Reihe von Reaktionen, die mit einer biologischen Komponente stattfinden, die Leitfähigkeit am Ort der biologischen Komponente ändert. Da diese biologischen Komponenten in der Regel auf den Elektroden immobilisiert werden, geht es um Leitfähigkeitsänderungen in der Grenzschicht Elektrode/Lösung und nicht unbedingt um solche, die auch makroskopisch als Änderung in der gesamten Lösung meßbar wären.

Wie bereits oben erwähnt wurde, sind die bisher bekannten Dünnschichtverfahren zur Herstellung von Biosensoren zu aufwendig und daher zu kostenintensiv.

Daher ist es Aufgabe der vorliegenden Erfindung, einen Leitfähigkeits-Biosensor bereit zu stellen, der einfach und stengünstig in der Herstellung ist.

Diese Aufgabe wird mit den im Kennzeichen des Anspruchs 1 befindlichen Merkmale erfindungsgemäß gelöst.

Demnach ist das Verfahren zur Herstellung von mehrdimensionalen Leitfähigkeits-Biosensoren auf einem Substrat dadurch gekennzeichnet, daß auf das Substrat Pasten im Siebdruckverfahren als Dickschicht-Leitfähigkeitselektroden aus geeigneten Materialen aufgebracht werden und auf den Dickschicht-Leitfähigkeitselektroden Enzyme immobilisiert werden.

Mit dem hier vorgestellten Biosensor wurde gezeigt, daß auch Siebdruckverfahren zur Herstellung von Leitfähigkeitselektroden geeignet sind. Mit Hilfe dieser Verfahren können die gleichen Strukturen produziert werden wie mit Dünnschichtverfahren. Die Dickschicht-Technik hat den entscheidenden Vorteil, daß sie wesentlich preiswerter ist als die vorbekannte Dünnschicht-Technik, die nicht in jedem mittelständischen Betrieb Anwendung finden kann.

Außerdem ist es vorteilhaft, daß die Herstellung des Transducers mit der gleichen Technik erfolgen kann wie das Aufbringen der Enzymschicht.

Zur Herstellung von Dickschicht-Biosensoren lassen sich unterschiedliche Substrate und Pasten verwenden: Der klassische Dickschicht-Prozess beruht auf Pasten, die nach dem Drucken und Trocknen durch ein charakteristisches Temperaturprogramm eingebrannt werden, das durch eine Spitzentemperatur von 850 °C gekennzeichnet ist. Als Substrate dienen in diesem Falle Keramiken, aber auch andere Materialien können hierbei verwendet werden.

Neben den klassischen Materialien, wie Gold oder Platin, mit denen im wesentlichen zwei-dimensionale Strukturen aufgebaut werden können, ist seit einigen Jahren ungebrannte Keramik als Substratmittel eingeführt. Dieses Material hat den Vorteil, daß hier mehrere Lagen miteinander verpreßt und anschließend gebrannt werden können, so daß drei-dimensionale Strukturen mühelos erzielbar sind. Außerdem wurden Dickschichtpasten entwickelt, die bei Raumtemperatur aushärtbar sind, so daß als Substrat auch Kunststoffe eingesetzt werden können. Alle Substrate sind mit den entsprechenden Pasten zur Fertigung von Leitfähigkeits-Biosensoren geeignet.

Zur Herstellung zwei-dimensionaler Strukturen werden auf das Substrat zwei Elektroden mit einer Edelmetallpaste gedruckt. Als Metalle sind vor allem Platin und Gold geeignet. Die Zuleitungen leitungen können vorteilhafterweise mit einer anderen Pasten gedruckt werden, die z.B. Silber und/oder Palladium enthalten kann. Sie werden mit einer Isolierpaste abgedeckt.

Erfindungsgemäß wird auf den in Dickschicht-Technik aufgebrachten Leitfähigkeitselektroden ein Enzym immobilisiert, das eine Reaktion katalysiert, bei der aus neutralen Molekülen Ionen gebildet werden. Als Beispiele sind Urease, Penicillinase, Esterasen, Hydrolasen, Aminosäureoxidase und Glucoseoxidase zu nennen. Die Immobilisierung kann nach einer aus der Literatur bekannten Methode erfolgen, d.h. es kann sich um Adsorption, Einschluß in eine Matrix (z.B. Siebdrucktechnik), Quervernetzen mit bifunktionellen Reagenzien oder kovalente Anbindung an einen Träger handeln. Der so hergestellte Dickschicht-Leitfähigkeits-Biosensor kann zur Bestimmung des jeweiligen substrates oder aber zur Bestimmung der Enzymaktivität herangezogen werden. Über die Messung der Enzymaktivität können Inhibitoren oder Aktivatoren des Enzyms nachgewiesen werden. Vorteilhafterweise lassen sich drei-dimensionale Strukturen mit Hilfe der ungebrannten Keramik herstellen. D.h., daß sich durch Variation der Zahl der bedruckten Schichten Sensoren mit mindestens zwei Elektroden oder solche mit vier Elektroden bei sog. Vier-Pol-Messungen oder gar Mehrelektroden bei Mehrfachsensoren herstellen.

Weitere vorteilhafte Ausgestaltungen sind den Unteransprüchen und der Beschreibung der Ausführungsbeispiele zu entnehmen.

Die Erfindung wird nunmehr anhand der Zeichnungen im Detail erläutert. Es zeigen

Fig. 1      eine schematische Darstellung eines Dickschicht-Leitfähigkeitssensors mit zwei-dimensionaler Struktur;

Fig. 2      eine schematische Darstellung eines Dickschicht-Leitfähigkeitssensors als alternatives Layout zu dem aus Fig. 1;

Fig. 3      eine schematische Darstellung eines Dickschicht-Leitfähigkeitssensors als alternatives Layout zu Fig. 1 und 2;

Fig. 4      eine schematische Darstellung eines Leitfähigkeits-Dickschichtsensors mit drei-dimensionaler Struktur in Seitenansicht und die Ansicht der Stirnseite;

Fig. 5a      die Abhängigkeit der Admittanz von der KCl-Konzentration, die mit Hilfe eines Dickschicht-Leitfähigkeitssensors mit zwei-dimensionaler Struktur gewonnen wurde;

Fig. 5b      eine Harnstoff-Kalibrierkurve, die mit einem Dickschicht-Leitfähigkeitssensor mit zwei-dimensionaler Struktur und Uraesemembran gemessen wurde;

Fig. 6      eine Harnstoff-Kalibrierkurve die mit einem Dickschicht-Leitfähigkeitssensor mit drei-dimensionaler Struktur und Uraesemembran gemessen wurde.

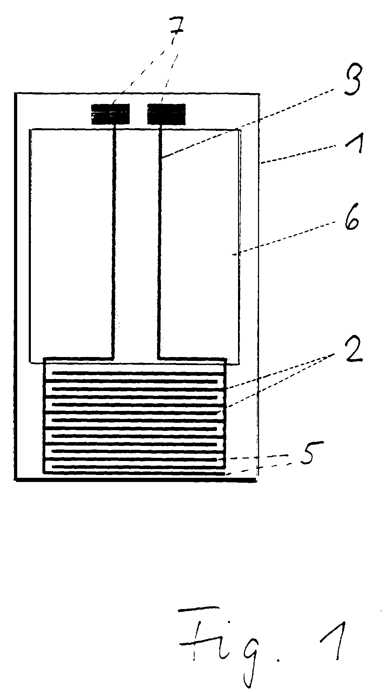

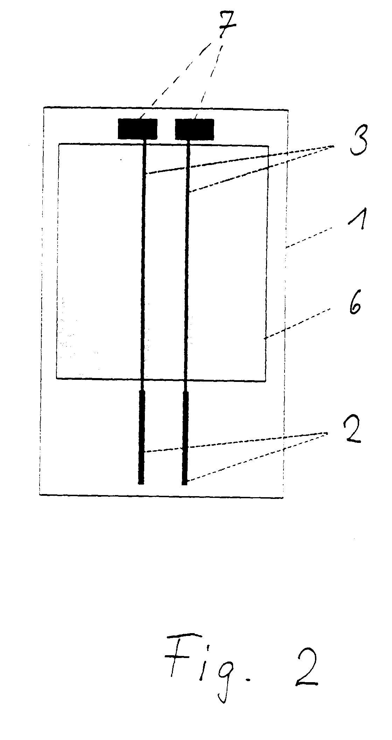

In Fig. 1 ist ein mögliches Layout auf einem Substrat 1 gezeigt. Es handelt sich hier um eine kammartige Struktur der erfindungsgemäßen Dickschicht-Leitfähigkeitselektroden 2, die mit einem Abstand von 300 μm und einer Elektrodenbreite von 500 μm angeordnet sind. Die gesamte aktive Elektrodenfläche beträgt etwa 1,7 cm$^2$. Sie kann jedoch ohne Veränderung des Ergebnisses variiert werden, indem die Zahl der "Zinken" 5 der Kämme verändert wird. Ebenso tritt keine Veränderung des Ergebnisses ein, wenn der Abstand oder die Breite der Zinken 5 verändert wird. Als Extremfall dieser Abwandlungen kann ein Layout mit zwei erfindungsgemäßen parallelen Dickschicht-Leitfähigkeitselektroden 2 betrachtet werden, wie es der Fig. 2 zu entnehmen ist.

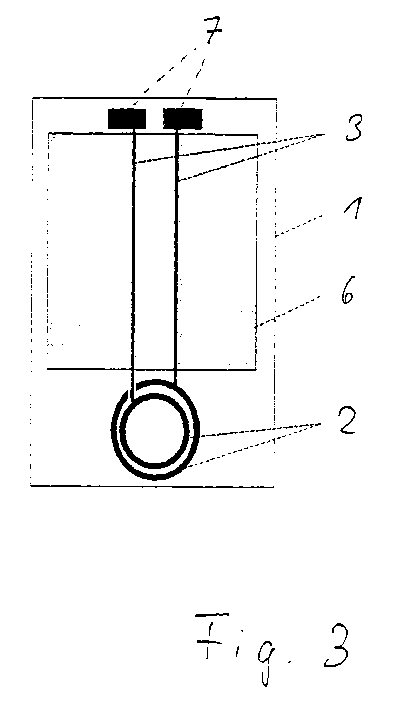

Aber auch grundsätzlich andere Anordnungen an Elektroden sind möglich und können zur Messung der Leitfähigkeit eingesetzt werden. Als Beispiel wäre noch die Anordnung der erfindungsgemäßen Dickschicht-Leitfähigkeitselektroden 2 in konzentrischen Kreisen zu nennen, so wie es in Fig. 3 gezeigt wird.

Widerstandsmessungen sind nicht nur mit zwei Elektroden, sondern auch mit vier Elektroden möglich, d.h. bei einer sog. 4-Pol-Messung, die in mehr als zweidimensionalen Strukturen Anwendung findet. Gerade diese mehrdimensionalen Strukturen sind für die erfindungsgemäßen Biosensoren besonders gut geeignet und einfach herzustellen.

Weiterhin sind in den Fig. 1 bis 3 die Leiterbahnen 3 zu erkennen, die als Stromzuführungsleitungen für die Leitfähigkeitsmessung dienen. Diese Leiterbahnen werden über eine Isolierschicht 6 geführt, die zwischen den Anschlüssen 7 und den eigentlichen Dickschicht-Leitfähigkeitselektroden auf das Substrat 1 aufgebracht ist.

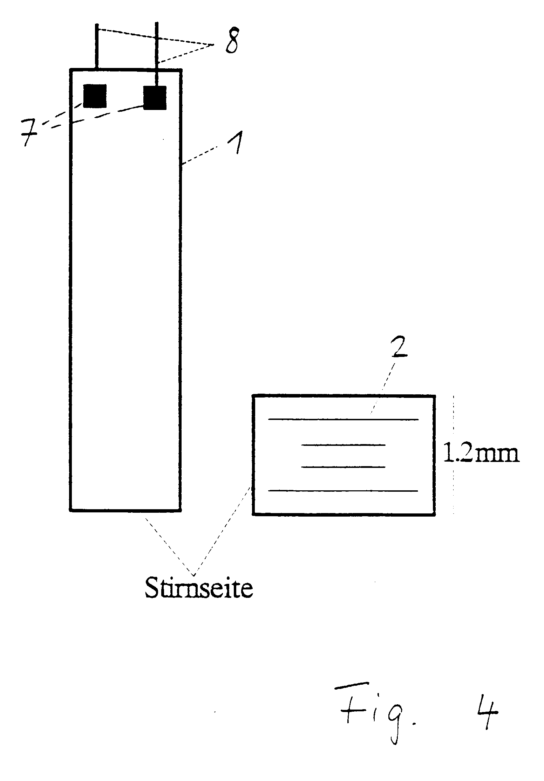

In Fig. 4 ist eine schematische Darstellung eines Dickschichtsensors mit drei-dimensionaler Struktur gezeigt. Die Figur zeigt ferner die Seitenansicht und die Ansicht einer Stirnfläche dieses drei-dimensionalen Dickschichtsensors. Hier sind die Dickschicht-Leitfähigkeitselektroden 2 in mehreren Substratschichten übereinander zusammengebracht, so daß sich insgesamt eine Höhe der Schichten von 1,2 mm ergibt. Die Ableitungen 8 sind jeweils auf einer Seitenfläche des Biosensors angebracht und zwar dort, wo sich die Anschlüsse 7 der Leiterbahnen 3 befinden.

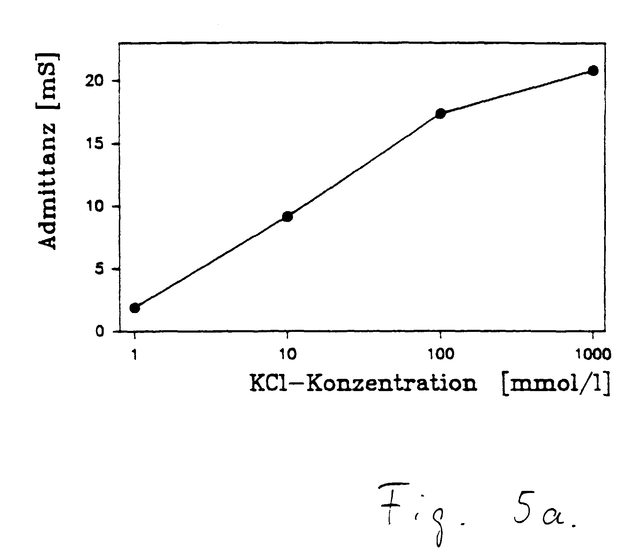

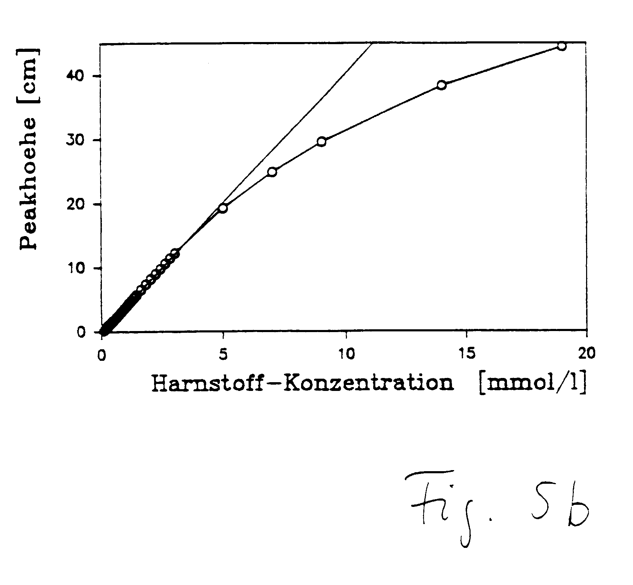

In den Fig. 5a und 5b sind Kalibrierkurven gezeigt, die mit den erfindungsgemäßen Dickschicht-Leitfähigkeitselektroden erzielt wurden. In Fig. 5a ist die Admittanz in Abhängigkeit von der KCl-Konzentration bei einer Wechselspannungsfrequenz von 1kHz und einer Amplitude von 100 mV aufgetragen. Damit ist die Eignung dieser Sensoren zur Bestimmung der Leitfähigkeit von Elektrolytlösungen gezeigt. Zur Bestimmung von Harnstoff wurde nicht die Admittanz Y direkt aufgezeichnet, sondern ihre Änderungsgeschwindigkeit (dY/dt) bei Zugabe von Harnstoff zu einer Harnstoff-freien Lösung.

Auf diese Weise kann die Meßdauer erheblich abgekürzt werden, da keine Gleichgewichtseinstellung abgewartet werden muß. Als Signal erhält man typischerweise Peaks, deren Höhe ausgewertet wird.

In Fig. 5b ist die Peakhöhe in Abhängigkeit von der Harnstoff-Konzentration aufgetragen. Dieser Sensor wurde aus dem von Fig. 5a erhalten. Der Biosensor für diese Messung war von der gleichen Art, wie er in Fig. 5a benutzt wurde, jedoch auf den Elektroden das Enzym Urease durch Quervernetzen mit dem bifunktionellen Reagenz Glutaraldehyd immobilisiert wurde.

Urease katalysiert die Hydrolyse von Harnstoff:

$$\text{Harnstoff} + 3\,H_2O \text{-----} 2\,NH_4^+ + HCO_3^- + OH^-$$

Durch geeignete Kopplung mehrerer Enzyme auf den Elektroden sind außer den oben erwähnten Enzymsubstraten weitere einer Messung mit Leitfähigkeitselektroden zugänglich, z.B. kann durch Kopplung von Creatinase und Urease Creatin und durch Kopplung von Creatininase, Creatinase und Urease auch Creatinin bestimmt werden:

Creatininase: $Creatinin + H_2O \text{-----} Creatin^- + H^+$
Creatin: $Creatin^- + H_2O \text{-----} Sarcosin^{2-} + Harnstoff + H^+$
Urease: $Harnstoff + 3\,H_2O \text{-----} 2\,NH_4^+ + HCO_3H^- + OH^-$

Auf diese Weise können auf einem Dickschichtsubstrat Sensoren für mehrere Analyte kombiniert werden.

Eine drei-dimensionale Struktur, wie sie in Fig. 4 gezeigt ist, läßt sich z.B. mit Hilfe der ungebrannten Keramik herstellen. Durch Variation der Zahl der bedruckten Schichten lassen sich Sensoren mit mindestens zwei Elektroden oder mehr Elektroden (Mehrfachsensoren) herstellen.

Als Enzyme können die gleichen wie die oben erwähnt eingesetzt werden, die auf der Stirnseite des Sensors immobilisiert werden. Als Methoden zur Immobilisierung sind die in der Literatur beschriebenen Methoden geeignet, insbesondere das Quervernetzen des Enzyms mit Glutaraldehyd.

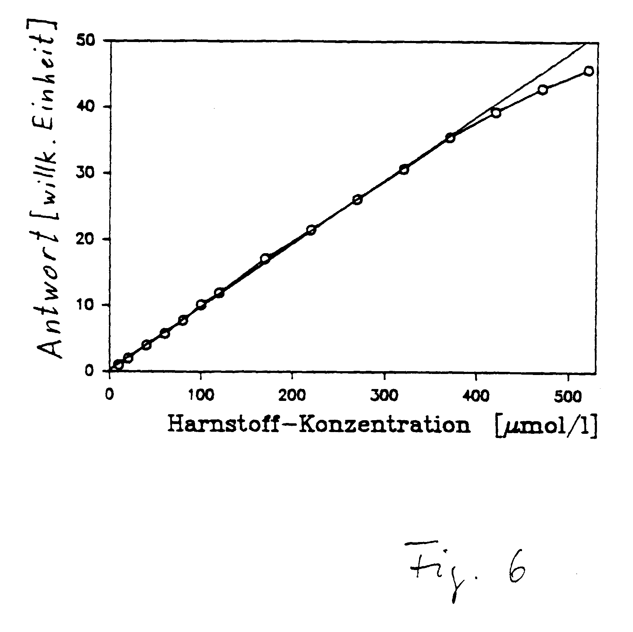

Die Fig. 6 zeigt als Beispiel eine Kalibrierkurve für Harnstoff. Der Biosensor wurde durch Quervernetzen von Urease mit Glutaraldehyd auf einem Sensor mit dem Design von Fig. 4 hergestellt. Die Messungen wurden mit einer Wechselspannung von 4 kHz und einer Amplitude von 1 V durchgeführt.

Mit der vorliegenden Erfindung wurde somit gezeigt, daß es möglich ist, mit der sog. Dickschicht-Technik Biosensoren herzustellen, die einerseits einfacher in der Herstellung und andererseits wesentlich kostengünstiger sind.

**Patentansprüche**

1. Verfahren zur Herstellung von Leitfähigkeits-Biosensoren, dadurch **gekennzeichnet,** daß

   - auf ein Substrat (1) Pasten im Siebdruckverfahren als Dickschicht-Leitfähigkeitselektroden aus geeigneten Materialien aufgebracht werden; und
   - auf die Dickschicht-Leitfähigkeitselektroden (2) Enzyme immobilisiert werden.

2. Verfahren nach Anspruch 1, dadurch **gekennzeichnet,** daß als Substrat (1) ungebrannte Keramik verwendet wird.

3. Verfahren nach Anspruch 1, dadurch **gekenn-**

**zeichnet,** daß als Substratmaterialien gebrannte Keramik, Kunststoffe und andere Dickschichtmaterialien verwendet werden.

4. Verfahren nach Anspruch 1, **dadurch gekennzeichnet,** daß für die Pasten zur Herstellung der Dickschicht-Leitfähigkeitselektroden (2) auf den Substraten (1) Edelmetalle verwendet werden.

5. Verfahren nach Anspruch 1, dadurch **gekennzeichnet,** daß für die Zuleitungen (3) zu den Dickschicht-Leitfähigkeitselektroden (2) andere leitfähige Pasten verwendet werden können, als die zur Herstellung der Dickschicht-Leitfähigkeitselektroden (2).

6. Verfahren nach Anspruch 1 und 2, dadurch **gekennzeichnet,** daß die Zahl der in der Dickschicht-Technik bedruckten Substratschichten (1) variiert wird.

7. Verfahren nach Anspruch 1 und 2, dadurch **gekennzeichnet,** daß mehrere Schichten (1) miteinander verpreßt und anschließend gebrannt werden.

8. Verfahren nach Anspruch 1, dadurch **gekennzeichnet,** daß auf die Dickschicht-Leitfähigkeitselektroden (2) ein Enzym immobilisiert wird, das eine Reaktion katalysiert, so daß aus neutralen Molekülen Ionen gebildet werden.

9. Verfahren nach Anspruch 3, dadurch **gekennzeichnet,** daß auf die Dickschicht-Leitfähigkeitselektroden (2) durch geeignete Kopplung mehrere Enzyme aufgebracht werden.

10. Verfahren nach einem der vorangegangenen Ansprüche, dadurch **gekennzeichnet,** daß auf ein Substrat (1) Sensoren für mehrere Analyte kombiniert werden.

11. Verfahren nach einem der vorangegangenen Ansprüche, dadurch **gekennzeichnet,** daß die Herstellung eines Transducers mit der gleichen Technik (Siebdruck) wie das Aufbringen der Enzymschicht erfolgt.

12. Verfahren nach Anspruch 1, dadurch **gekennzeichnet,** daß ein Enzym mit einem bifunktionellen Reagenz durch Quervernetzung immobilisiert wird.

13. Leitfähigkeits-Biosensoren, bestehend aus einem Substrat auf dem sich Elektroden befinden, dadurch **gekennzeichnet**, daß

- die Elektroden auf dem Substrat (1) als Dickschicht-Leitfähigkeitselektroden (2) aus einer Paste geeigneten Materials bestehen, die im Siebdruckverfahren auf das Substrat (1) aufgebracht sind; und

- an der Oberfläche der Dickschicht-Leitfähigkeitselektroden (2) Enzyme immobilisiert sind.

14. Leitfähigkeits-Biosensoren nach Anspruch 13, dadurch **gekennzeichnet**, daß die Dickschicht-Leitfähigkeitselektroden (2) in verschiedenen Formen auf das Substrat (1) aufgebracht sind.

15. Leitfähigkeits-Biosensoren nach Anspruch 13 und 14, dadurch **gekennzeichnet,** daß die Dickschicht-Leitfähigkeitselektroden (2) parallele Linien darstellen.

16. Leitfähigkeits-Biosensoren nach Anspruch 13 und 14, dadurch **gekennzeichnet**, daß die Dickschicht-Leitfähigkeitselektroden (2) eine kammartige Struktur aufweisen.

17. Leitfähigkeits-Biosensoren nach Anspruch 13 und 14, dadurch **gekennzeichnet**, daß die Dickschicht-Leitfähigkeitselektroden (2) konzentrische Kreise darstellen.

18. Leitfähigkeits-Biosensoren nach Anspruch 13, dadurch **gekennzeichnet**, daß auf die Dickschicht-Leitfähigkeitselektroden (2) ein Enzym mit einem bifunktionellen Reagenz durch Quervernetzung immobilisiert wird.

19. Leitfähigkeits-Biosensoren nach Anspruch 13, dadurch **gekennzeichnet**, daß auf die Dickschicht-Leitfähigkeitselektroden (2) ein Enzym mit der Siebdrucktechnik immobilisiert wird.

20. Leitfähigkeits-Biosensoren nach einem der Ansprüche 13 bis 19, **dadurch gekennzeichnet**, daß mehrere Substratschichten übereinander angeordnet sind, so daß ein mehrdimensionaler Biosensor entsteht.

**Claims**

1. Method for the production of conductivity biosensors, characterised in that

• pastes are deposited by the screen-printing method on a substrate (1) as thick-film conductivity electrodes of suitable materials; and
• enzymes are immobilised on the thick-film conductivity electrodes (2).

2. Method according to claim 1, characterised in that unfired ceramic is used as a substrate (1).

3. Method according to claim 1, characterised in that fired ceramic, plastics and other thick-film materials are used as substrate materials.

4. Method according to claim 1, characterised in that noble metals are used for producing the thick-film conductivity electrodes (2) on the substrates (1).

5. Method according to claim 1, characterised in that conductive pastes can be used for the supply conductors (3) to the thick-film conductivity electrodes (2) other than those for producing the thick-film conductivity electrodes (2).

6. Method according to claim 1 and 2, characterised in that the number of substrate layers (1) printed using thick-film technology is varied.

7. Method according to claim 1 and 2, characterised in that a plurality of layers (1) are pressed together and then fired.

8. an enzyme is immobilised on the thick-film conductivity electrodes (2), said enzyme catalysing a reaction so that ions are formed from neutral molecules.

9. Method according to claim 3, characterised in that a plurality of enzymes are applied to the thick-film conductivity electrodes (2) by appropriate coupling.

10. Method according to one of the preceding claims, characterised in that sensors for a plurality of analytes are combined on one substrate (1).

11. Method according to one of the preceding claims, characterised in that the production of a transducer is effected by the same technique (screen printing) as the application of the enzyme layer.

12. Method according to claim 1, characterised in that an enzyme with a bifunctional reagent is immobilised by cross-linking.

13. Conductivity biosensors, comprising a substrate upon which electrodes are located, characterised in that

- the electrodes on the substrate as thick-film conductivity electrodes (2) are composed of a paste of suitable material which are applied to the substrate (1) by the screen-printing method, and
- enzymes are immobilised on the surface of the thick-film conductivity electrodes (2).

14. Conductivity biosensors according to claim 13, characterised in that the thick-film conductivity electrodes (2) are applied to the substrate (1) in various forms.

15. Conductivity biosensors according to claim 13 and 14, characterised in that the thick-film conductivity electrodes (2) represent parallel lines.

16. Conductivity biosensors according to claim 13 and 14, characterised in that the thick-film conductivity electrodes (2) have a comb-like structure.

17. Conductivity biosensors according to claim 13 and 14, characterised in that the thick-film conductivity electrodes (2) represent concentric circles.

18. Conductivity biosensors according to claim 13, characterised in that an enzyme with a bifunctional reagent is immobilised by cross-linking on the thick-film conductivity electrodes (2).

19. Conductivity biosensors according to claim 13, characterised in that en enzyme is immobilised on the thick-film conductivity electrodes (2) by the screen-printing technique.

20. Conductivity biosensors according to one of claims 13 to 19, characterised in that a plurality of substrate layers are disposed one above the other, so that a multidimensional biosensor results.

## Revendications

1. Procédé de préparation de biocapteurs conductimétriques, caractérisé en ce que l'on applique sur un substrat (1), par sérigraphie, des pâtes d'un matériau approprié sous forme d'électrodes conductimétriques en couche épaisse, et en ce que l'on immobilise des enzymes sur les électrodes conductimétriques en couche épaisse (2).

2. Procédé conforme à la revendication 1, caractérisé en ce que l'on utilise, comme substrat (1), de la céramique non cuite.

3. Procédé conforme à la revendication 1, caractérisé en ce que l'on utilise, comme matériaux formant le substrat, de la céramique non cuite, des matières plastiques et d'autres matériaux pour couches épaisses.

4. Procédé conforme à la revendication 1, caractérisé en ce que l'on utilise des métaux nobles pour les pâtes destinées à la préparation des électrodes conductimétriques en couches épaisses (2) sur les substrats (1).

5. Procédé conforme à la revendication 1, caractérisé en ce que l'on peut utiliser pour les fils conducteurs (3) arrivant aux électrodes conductimétriques en couche épaisse (2) des pâtes conductrices différentes de celles utilisées pour la préparation des électrodes conductimétriques en couche épaisse.

6. Procédé conforme à la revendication 1 et 2, caractérisé en ce que l'on fait varier le nombre de couches de subtrat (1) imprimées selon la technique de dépôt de couches épaisses.

7. Procédé conforme à la revendication 1 et 2, caractérisé en ce que l'on assemble plusieurs couches de substrat (1) en les pressant les unes contre les autres, et en ce qu'on les soumet ensuite à une cuisson.

8. Procédé conforme à la revendication 1, caractérisé en ce que l'on immobilise sur les électrodes conductimétriques en couche épaisse (2) une enzyme qui catalyse une réaction entraînant la formation d'ions à partir de molécules neutres.

9. Procédé conforme à la revendication 3, caractérisé en ce que l'on applique, par un couplage approprié, plusieurs types d'enzymes sur les électrodes conductimétriques en couche épaisse (2).

10. Procédé conforme à une des revendications précédentes, caractérisé en ce que l'on réunit, sur un même substrat (1), plusieurs capteurs spécifiques de plusieurs substances à détecter.

11. Procédé conforme à une des revendications précédentes, caractérisé en ce que l'on effectue la préparation d'un transducteur selon la même technique (sérigraphic) que celle utilisée pour l'application de la couche d'enzymes.

12. Procédé conforme à la revendication 1, caractérisé en ce que l'on immobilise une enzyme par réticulation à l'aide d'un réactif bifonctionnel.

13. Biocapteurs conductimétriques comprenant un substrat portant des électrodes, caractérisés en ce que

- les électrodes sur le substrat (1) sont des électrodes conductimétriques en couches épaisses (2) formées à partir d'une pâte d'un matériau approprié, lesquelles couches sont appliquées par sérigraphie sur le substrat (1), et
- des enzymes sont immobilisées à la surface des électrodes conductimétriques en couche épaisse (2),

14. Biocapteurs conductimétriques conformes à la revendication 13, caractérisés en ce que les électrodes conductimétriques en couche épaisse (2) sont appliquées sur le substrat (1) sous différentes formes.

15. Biocapteurs conductimétriques conformes à la revendication 13 et 14, caractérisés en ce que les électrodes conductimétriques en couche épaisse (2) forment des lignes parallèles.

16. Biocapteurs conductimétriques conformes à la revendication 13 et 14, caractérisés en ce que les électrodes conductimétriques en couche épaisse (2) ont une structure en forme de peigne.

17. Biocapteurs conductimétriques conformes à la revendication 13 et 14, caractérisés en ce que les électrodes conductimétriques en couche épaisse (2) forment des cercles concentriques.

18. Biocapteurs conductimétriques conforme à la revendication 13, caractérisés en ce que l'on immobilise sur les électrodes conductimétriques en couche épaisse (2) une enzyme par réticulation à l'aide d'un réactif bifonctionnel.

19. Biocapteurs conductimétriques conformes à la revendication 13, caractérisés en ce que l'immobilisation d'une enzyme sur les électrodes conductimétriques en couche épaisse (2) se fait par sérigraphie.

20. Biocapteurs conductimétriques conformes à une des revendications 13 à 19, caractérisés en ce que plusieurs couches de substrat sont disposées les unes sur les autres formant ainsi un biocapteur à plusieurs dimensions.

Fig. 1

Fig. 2

Fig. 3

Stirnseite

1.2mm

Fig. 4

Fig. 5a.

Fig. 5b

Fig. 6